# EUROPEAN PATENT APPLICATION

(11) **EP 4 674 854 A1**
(43) Date of publication of application: **07.01.2026**
(21) Application number: 24763119.5
(22) Date of filing: 27.02.2024
(51) Int. Cl.: C07D 519/00, A61P 35/04, A61P 35/02, A61P 35/00, A61K 31/5383

(54) **TRIAZOLO RING COMPOUND METHANESULFONATE CRYSTAL FORM, PREPARATION METHOD THEREFOR AND USE THEREOF**

(30) Priority: 27.02.2023 CN 202310179589
(71) Applicant: Jiangsu Hansoh Pharmaceutical Group Co., Ltd., Lianyungang, Jiangsu 222047 (CN)
(72) Inventor: LIU, Mengyu, Lianyungang, Jiangsu 222047 (CN); LIAN, Xiaogang, Lianyungang, Jiangsu 222047 (CN); HE, Lei, Lianyungang, Jiangsu 222047 (CN)
(74) Representative: Cabinet Beau de Loménie
(86) International application number: PCT/CN2024/078701
(87) International publication number: WO 2024/179438

(57) **Abstract**

Disclosed in the present invention are a triazolo ring compound methanesulfonate crystal form, a preparation method therefor and a use thereof. Specifically, disclosed are a crystal form of a compound methanesulfonate of formula (I), and a preparation method therefor and a use thereof, wherein the methanesulfonate crystal form is selected from crystal forms A-I; and disclosed are a preparation method for the crystal form and a pharmaceutical composition containing the crystal form. The preparation method of the present invention is simple and easy to implement and suitable for industrial large-scale production; the crystal forms A-I of the compound methanesulfonate of formula (I) prepared have good solubility and stability, thereby facilitating the preparation and storage of pharmaceutical preparations.

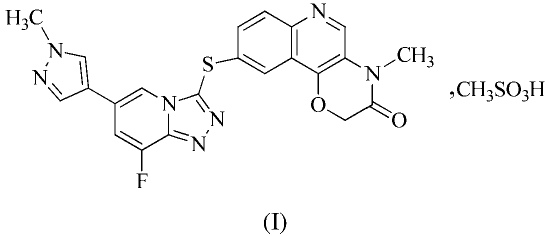

## Description

### Technical Field

The present invention belongs to the field of biomedicine, and in particular relates to a 9-((8-fluoro-6-(1-methyl-1*H*-pyrazol-4-yl)-[1,2,4]triazolo[4,3-*a*]pyridin-3-yl)-thio)-4-methyl-2*H*-[1,4]oxazino[3,2-c]quinoline-3(4*H*)-one methanesulfonate, a crystal form of the salt, a preparation method therefor and the use thereof.

### Background Art

Compound of formula (I), Chinese name: 9-((8- 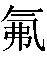 -6-(1- 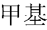 -1*H*- 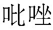 -4- 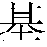 )- [1,2,4] 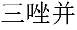 [4,3-*a*] 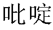 -3- 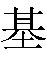 )- 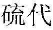 )-4- 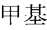 -2*H*-[1,4] 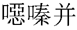 [3,2-*c*] 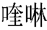 -3(4*H*)- 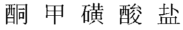 , English name: 9-((8-fluoro-6-(1-methyl-1H-pyrazol-4-yl)-[1,2,4]triazolo[4,3-a]pyridin-3-yl)-thio)-4-methyl-2H-[1,4]oxazino[3,2-c]quinoline-3(4H)-one methanesulfonate, having the following structural formula:

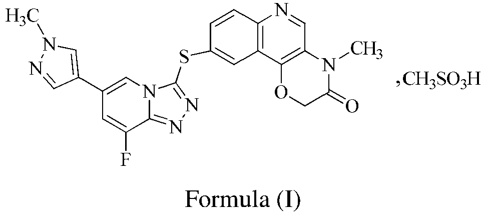

The hepatocyte growth factor (HFG) receptor, also known as C-Met, is a tyrosine kinase receptor. Abnormal activation of C-Met is associated with poor prognosis in cancer, and all involve the issue of C-Met overexpression. C-Met abnormalities are also found in various types of tumours, such as hepatocellular carcinoma (HCC), non-small cell carcinoma (NSCLC), bladder cancer, liver cancer, kidney cancer, breast cancer, squamous cell carcinoma, brain cancer, stomach cancer, colon cancer, etc. C-Met abnormalities can manifest as increased expression, gene amplification, gene mutation, or increased HGF expression. Under these abnormal conditions, C-Met can be in an abnormally activated state, resulting in carcinogenesis and poor prognosis. C-Met, upon abnormal activation, triggers tumour growth and neovascularization (angiogenesis, which can provide nutrients to the tumour), helping the spread of cancer to other organs (metastasis). Inhibition of the C-Met signalling pathway is therefore one of the important strategies for the treatment of tumours. The compound of formula I is a potent C-Met/HGFR (hepatocyte growth factor receptor) kinase inhibitor. As a tyrosine kinase inhibitor, the compound of formula I can effectively block the HGF/C-Met signalling pathway to achieve the purpose of treating abnormal cell growth (such as cancer) in mammals.

JIANGSU HANSOH PHARMACEUTICAL GROUP CO., LTD. discloses crystalline free bases of the compound of formula (I) or crystalline acid salts thereof, preparation methods therefor and uses thereof in patent CN 201580025022.6, in which methods for preparing crystal forms I, II, III, IV and V of the methanesulfonate, and pharmaceutical compositions containing same are disclosed.

### Summary of the Invention

The present invention aims to provide a crystal form of a compound methanesulfonate of formula (I) suitable for pharmaceutical development, a preparation method therefor and the use thereof. By means of in-depth study of different aggregation states of the methanesulfonate, nine polymorphs of the compound methanesulfonate of formula (I) are obtained.

An objective of the present invention is to provide 9-((8-fluoro-6-(1-methyl-1*H*-pyrazol-4-yl)-[1,2,4]triazolo[4,3-*a*]pyridin-3-yl)-thio)-4-methyl-2*H-*[1,4]oxazino[3,2-*c*]quinoline-3(4*H*)-one methanesulfonate, a compound of formula (I).

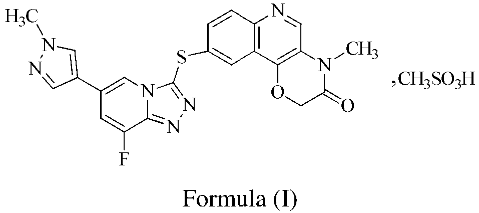

In a preferred embodiment of the present invention, the compound methanesulfonate of formula (I) is crystalline or amorphous.

In a preferred embodiment of the present invention, the crystal form includes crystal form A, crystal form B, crystal form C, crystal form D, crystal form E, crystal form F, crystal form G, crystal form H and crystal form I.

The crystal form A of the compound methanesulfonate of formula (I) has an X-ray powder diffraction pattern having a diffraction peak at 2θ of 5.4 ± 0.2°, or having a diffraction peak at 6.1 ± 0.2°, or having a diffraction peak at 10.6 ± 0.2°, or having a diffraction peak at 13.7 ± 0.2°, or having a diffraction peak at 18.1 ± 0.2°, or having a diffraction peak at 19.6 ± 0.2°, or having a diffraction peak at 24.7 ± 0.2°, preferably comprising any 2 to 5, or 3 to 5, or 3 to 6, or 3 to 7 of the above diffraction peaks, more preferably comprising any 4, 6, or 7 of the above diffraction peaks.

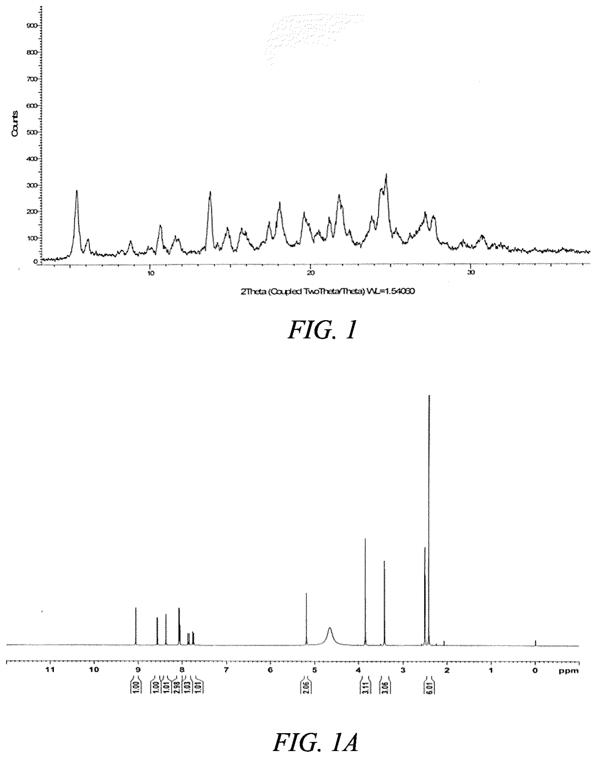

Preferably, the crystal form A has an X-ray powder diffraction pattern having diffraction peaks at 2θ of 5.4 ± 0.2°, 6.1 ± 0.2°, 10.6 ± 0.2° and 13.7 ± 0.2°, preferably further comprising diffraction peaks at 2θ of 18.1 ± 0.2°, 19.6 ± 0.2° and 24.7 ± 0.2°, more preferably further comprising diffraction peaks at 2θ of 8.7 ± 0.2°, 11.7 ± 0.2°, 14.8 ± 0.2°, 17.4 ± 0.2°, 21.1 ± 0.2° and 27.1 ± 0.2°; further preferably, the crystal form A has an X-ray powder diffraction pattern substantially as shown in FIG. 1 and a proton nuclear magnetic resonance spectrum as shown in FIG. 1A.

In a preferred embodiment of the present invention, the molar ratio of free base to methanesulfonate in the crystal form A is about 1 : 2, as shown by the proton nuclear magnetic resonance spectrum.

The crystal form B of the compound methanesulfonate of formula (I) has an X-ray powder diffraction pattern having a diffraction peak at 2θ of 5.4 ± 0.2°, or having a diffraction peak at 7.8 ± 0.2°, or having a diffraction peak at 10.6 ± 0.2°, or having a diffraction peak at 13.7 ± 0.2°, or having a diffraction peak at 14.4 ± 0.2°, or having a diffraction peak at 15.6 ± 0.2°, or having a diffraction peak at 19.7 ± 0.2°, preferably comprising any 2 to 5, or 3 to 5, or 3 to 6, or 3 to 7 of the above diffraction peaks, more preferably comprising any 4, 6, or 7 of the above diffraction peaks.

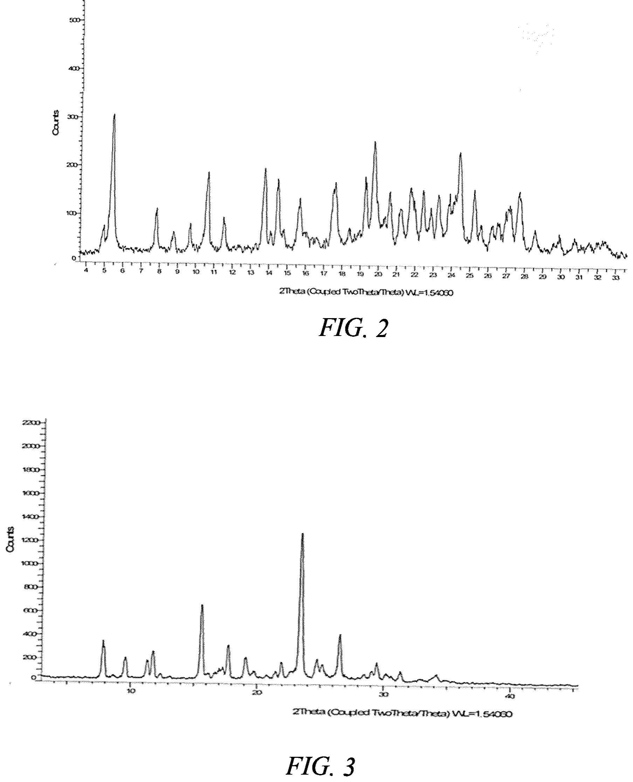

Preferably, the crystal form B has an X-ray powder diffraction pattern having diffraction peaks at 2θ of 5.4 ± 0.2°, 7.8 ± 0.2°, 10.6 ± 0.2° and 13.7 ± 0.2°, preferably further comprising characteristic peaks at 2θ of 14.4 ± 0.2, 15.6 ± 0.2 and 19.7 ± 0.2, more preferably further comprising diffraction peaks at 2θ of 4.9 ± 0.2°, 8.7 ± 0.2°, 9.7 ± 0.2°, 11.5 ± 0.2°, 17.6 ± 0.2°, 24.4 ± 0.2°, 25.2 ± 0.2° and 27.6 ± 0.2°; further preferably, the crystal form B has an X-ray powder diffraction pattern substantially as shown in FIG. 2.

The crystal form C of the compound methanesulfonate of formula (I) has an X-ray powder diffraction pattern having a diffraction peak at 2θ of 7.8 ± 0.2°, or having a diffraction peak at 9.6 ± 0.2°, or having a diffraction peak at 11.7 ± 0.2°, or having a diffraction peak at 15.6 ± 0.2°, or having a diffraction peak at 17.7 ± 0.2°, or having a diffraction peak at 23.4 ± 0.2, or having a diffraction peak at 26.5 ± 0.2, preferably comprising any 2 to 5, or 3 to 5, or 3 to 6, or 3 to 7 of the above diffraction peaks, more preferably comprising any 4, 6, or 7 of the above diffraction peaks.

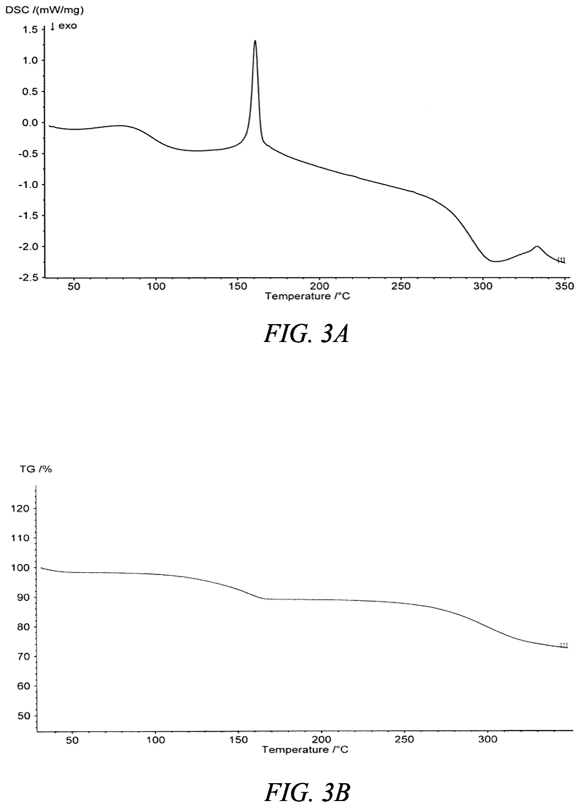

Preferably, the crystal form C has an X-ray powder diffraction pattern having diffraction peaks at 2θ of 7.8 ± 0.2°, 9.6 ± 0.2°, 11.7 ± 0.2° and 15.6 ± 0.2°, preferably further comprising characteristic peaks at 2θ of 17.7 ± 0.2, 23.4 ± 0.2 and 26.5 ± 0.2, more preferably further comprising diffraction peaks at 2θ of 11.3 ± 0.2°, 12.4 ± 0.2°, 19.1 ± 0.2°, 21.9 ± 0.2°, 24.6 ± 0.2°, 25.1 ± 0.2°, 29.4 ± 0.2°, 31.3 ± 0.2° and 34.2 ± 0.2°; further preferably, the crystal form C has an X-ray powder diffraction pattern substantially as shown in FIG. 3, a DSC pattern substantially as shown in FIG. 3A, and a TGA pattern is as shown in FIG. 3B; still further preferably, the crystal form C has a DSC pattern having a characteristic peak in the range of 50°C to 105°C and a characteristic peak in the range of 157°C to 164°C, or, the crystal form C has a TGA pattern showing a weight loss of 10% to 13% at 35°C to 165°C.

The crystal form D of the compound methanesulfonate of formula (I) has an X-ray powder diffraction pattern having a diffraction peak at 2θ of 5.2 ± 0.2°, or having a diffraction peak at 11.5 ± 0.2°, or having a diffraction peak at 13.5 ± 0.2°, or having a diffraction peak at 17.1 ± 0.2°, or having a diffraction peak at 19.8 ± 0.2, or having a diffraction peak at 22.8 ± 0.2, or having a diffraction peak at 23.3 ± 0.2, or having a diffraction peak at 24.2 ± 0.2, preferably comprising any 2 to 5, or 3 to 5, or 3 to 6, or 3 to 8 of the above diffraction peaks, more preferably comprising any 4, 6, or 8 of the above diffraction peaks.

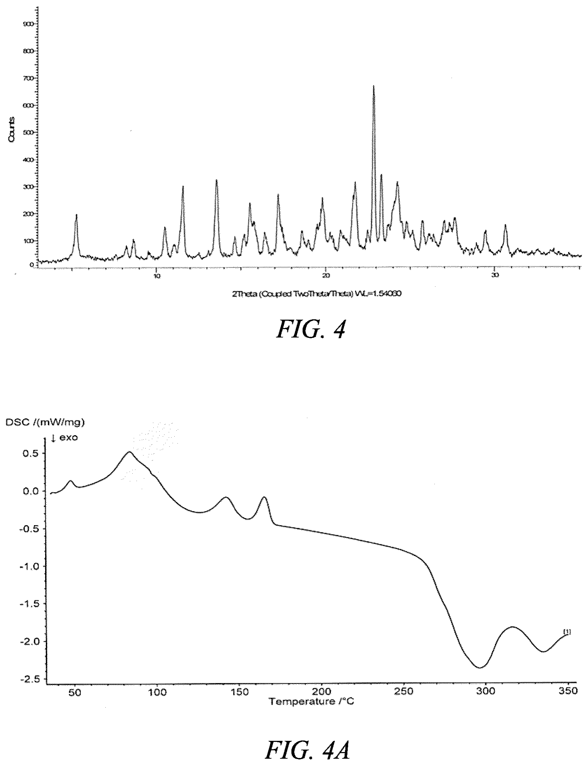

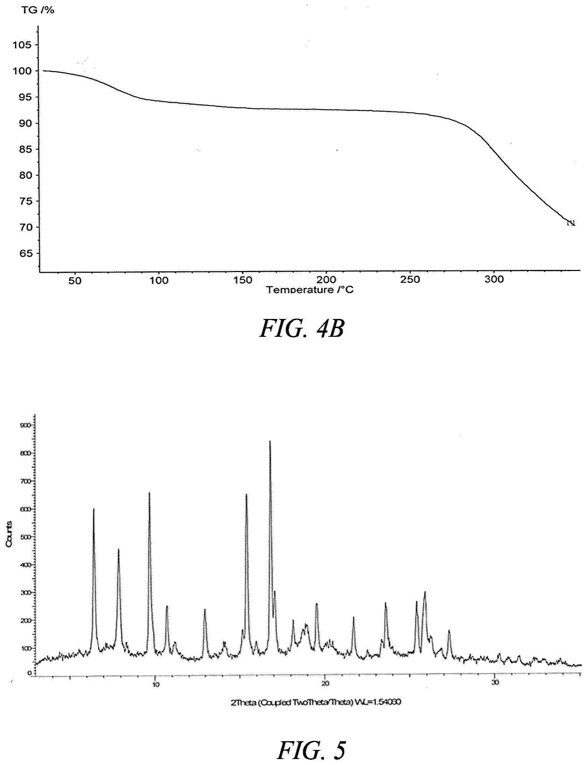

Preferably, the crystal form D has an X-ray powder diffraction pattern having diffraction peaks at 2θ of 5.2 ± 0.2°, 11.5 ± 0.2°, 13.5 ± 0.2° and 17.1 ± 0.2°, preferably further comprising characteristic peaks at 2θ of 19.8 ± 0.2, 22.8 ± 0.2, 23.3 ± 0.2 and 24.2 ± 0.2, more preferably further comprising diffraction peaks at 2θ of 8.2 ± 0.2°, 8.6 ± 0.2°, 10.5 ± 0.2°, 14.6 ± 0.2°, 16.4 ± 0.2°, 20.8 ± 0.2°, 21.7 ± 0.2°, 25.7 ± 0.2°, 29.4 ± 0.2° and 30.6 ± 0.2°; further preferably, the crystal form D has an X-ray powder diffraction pattern substantially as shown in FIG. 4, a DSC pattern substantially as shown in FIG. 4A, and a TGA pattern as shown in FIG. 4B; preferably, the crystal form D has a DSC pattern having a characteristic peak in the range of 71°C to 100°C, a characteristic peak in the range of 133°C to 155°C, and a characteristic peak in the range of 160°C to 170°C, or, the crystal form D has a TGA pattern showing a weight loss of 8% to 10% at 32°C to 105°C.

In a preferred embodiment of the present invention, the crystal form D is a methanesulfonate hydrate with a detected water content of 10.8%.

In a preferred embodiment of the present invention, the molar ratio of water to methanesulfonate in the crystal form D is about 3 : 1.

The crystal form E of the compound methanesulfonate of formula (I) has an X-ray powder diffraction pattern having a diffraction peak at 2θ of 6.4 ± 0.2°, or having a diffraction peak at 7.8 ± 0.2°, or having a diffraction peak at 9.7 ± 0.2°, or having a diffraction peak at 10.7 ± 0.2°, or having a diffraction peak at 12.9 ± 0.2°, or having a diffraction peak at 15.4 ± 0.2°, or having a diffraction peak at 16.8 ± 0.2°, or having a diffraction peak at 19.5 ± 0.2°, preferably comprising any 2 to 5, or 3 to 5, or 3 to 6, or 3 to 8 of the above diffraction peaks, more preferably comprising any 4, 6, or 8 of the above diffraction peaks.

Preferably, the crystal form E has an X-ray powder diffraction pattern having diffraction peaks at 2θ of 6.4 ± 0.2°, 7.8 ± 0.2°, 9.7 ± 0.2° and 10.7 ± 0.2°, preferably further comprising characteristic peaks at 2θ of 12.9 ± 0.2, 15.4 ± 0.2, 16.8 ± 0.2 and 19.5 ± 0.2, more preferably further comprising diffraction peaks at 2θ of 11.1 ± 0.2°, 14.1 ± 0.2°, 18.1 ± 0.2°, 21.6 ± 0.2°, 23.5 ± 0.2°, 25.4 ± 0.2° and 25.9 ± 0.2°; further preferably, the crystal form E has an X-ray powder diffraction pattern substantially as shown in FIG. 5.

The crystal form F of the compound methanesulfonate of formula (I) has an X-ray powder diffraction pattern having a diffraction peak at 2θ of 5.4 ± 0.2°, or having a diffraction peak at 6.5 ± 0.2°, or having a diffraction peak at 8.1 ± 0.2°, or having a diffraction peak at 9.7 ± 0.2°, or having a diffraction peak at 13.3 ± 0.2°, or having a diffraction peak at 15.5 ± 0.2°, or having a diffraction peak at 16.9 ± 0.2°, or having a diffraction peak at 21.6 ± 0.2°, preferably comprising any 2 to 5, or 3 to 5, or 3 to 6, or 3 to 8 of the above diffraction peaks, more preferably comprising any 4, 6, or 8 of the above diffraction peaks.

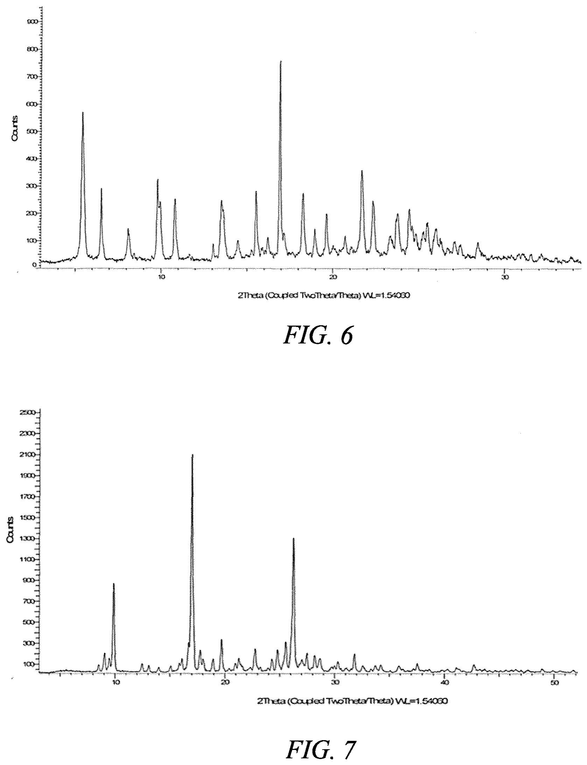

Preferably, the crystal form F has an X-ray powder diffraction pattern having diffraction peaks at 2θ of 5.4 ± 0.2°, 6.5 ± 0.2°, 8.1 ± 0.2° and 9.7 ± 0.2°, preferably further comprising characteristic peaks at 2θ of 13.3 ± 0.2, 15.5 ± 0.2°, 16.9 ± 0.2° and 21.6 ± 0.2°, more preferably further comprising diffraction peaks at 2θ of 10.8 ± 0.2°, 13.0 ± 0.2°, 18.2 ± 0.2°, 18.9 ± 0.2°, 19.6 ± 0.2°, 22.3 ± 0.2°, 23.7 ± 0.2° and 24.4 ± 0.2°; further preferably, the crystal form F has an X-ray powder diffraction pattern substantially as shown in FIG. 6.

The crystal form G of the compound methanesulfonate of formula (I) has an X-ray powder diffraction pattern having a diffraction peak at 2θ of 8.5 ± 0.2°, or having a diffraction peak at 9.0 ± 0.2°, or having a diffraction peak at 9.8 ± 0.2°, or having a diffraction peak at 16.9 ± 0.2°, or having a diffraction peak at 19.7 ± 0.2°, or having a diffraction peak at 22.7 ± 0.2°, or having a diffraction peak at 25.5 ± 0.2°, or having a diffraction peak at 26.2 ± 0.2°, or having a diffraction peak at 31.8 ± 0.2°, or having a diffraction peak at 32.5 ± 0.2°, preferably comprising any 2 to 5, or 3 to 5, or 3 to 6, or 3 to 8 of the above diffraction peaks, more preferably comprising any 4, 6, or 8 of the above diffraction peaks.

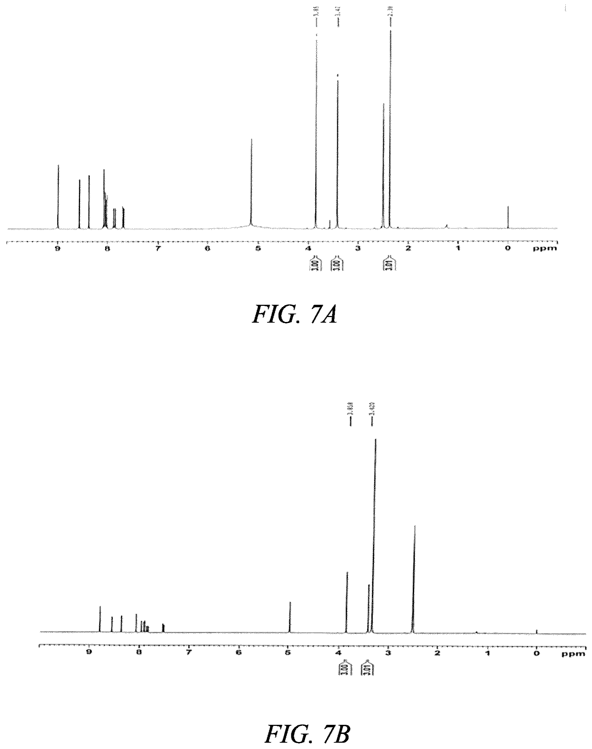

Preferably, the crystal form G has an X-ray powder diffraction pattern having diffraction peaks at 2θ of 9.8 ± 0.2°, 16.9 ± 0.2° and, preferably further comprising characteristic peaks at 2θ of 8.5 ± 0.2° and 9.0 ± 0.2°, preferably further comprising characteristic peaks at 2θ of 19.7 ± 0.2°, 22.7 ± 0.2°, 25.5 ± 0.2° and 26.2 ± 0.2°, more preferably further comprising diffraction peaks at 2θ of 9.4 ± 0.2°, 12.4 ± 0.2°, 13.0 ± 0.2°, 24.8 ± 0.2°, 27.4 ± 0.2°, 28.1 ± 0.2°, 31.8 ± 0.2° and 32.5 ± 0.2°; further preferably, the crystal form G has an X-ray powder diffraction pattern substantially as shown in FIG. 7 and a proton nuclear magnetic resonance spectrum as shown in FIG. 7A, and the free base of the crystal form G has a proton nuclear magnetic resonance spectrum as shown in FIG. 7B.

In a preferred embodiment of the present invention, the crystal form G is a methanesulfonate hydrate with water content of 5.0% to 8.0%.

In a preferred embodiment of the present invention, the crystal form G is a methanesulfonate hydrate with a detected water content of 6.0%.

In a preferred embodiment of the present invention, the molar ratio of water to methanesulfonate in the crystal form G is about 2 : 1.

In a preferred embodiment of the present invention, the molar ratio of free base to methanesulfonate in the crystal form G is about 1 : 1.

The crystal form G of the compound methanesulfonate of formula (I) prepared according to the present invention has the characteristics of simple preparation method, high crystal form purity and stable chemical state, which not only exhibits improved physicochemical properties of the crystalline free base of the compound of formula (I), but is also prone to polymorphic transformation and dissociation under high humidity conditions, making it highly suitable for pharmaceutical development.

The crystal form H of the compound methanesulfonate of formula (I) has an X-ray powder diffraction pattern having a diffraction peak at 2θ of 7.5 ± 0.2°, or having a diffraction peak at 8.1 ± 0.2°, or having a diffraction peak at 15.1 ± 0.2°, or having a diffraction peak at 17.1 ± 0.2°, or having a diffraction peak at 19.5 ± 0.2°, or having a diffraction peak at 22.7 ± 0.2°, or having a diffraction peak at 23.3 ± 0.2°, or having a diffraction peak at 30.5 ± 0.2°, preferably comprising any 2 to 5, or 3 to 5, or 3 to 6, or 3 to 8 of the above diffraction peaks, more preferably comprising any 4, 6, or 8 of the above diffraction peaks.

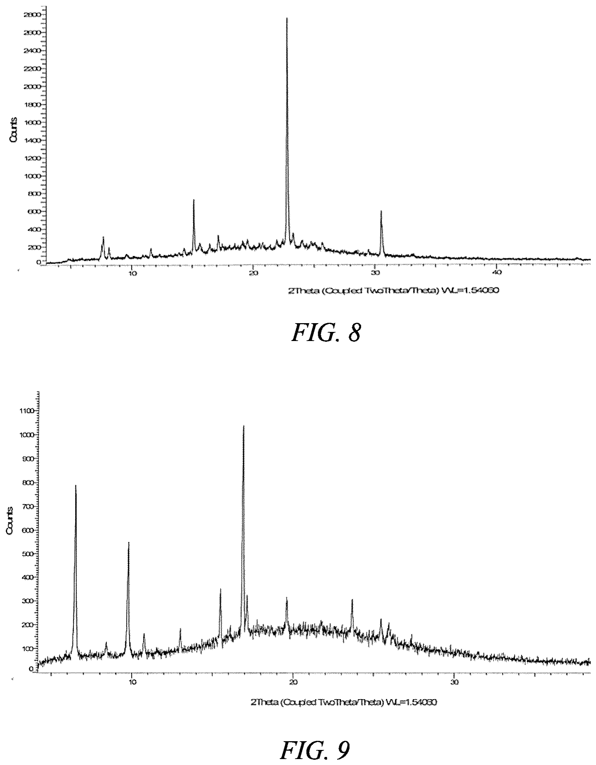

Preferably, the crystal form H has an X-ray powder diffraction pattern having diffraction peaks at 2θ of 7.5 ± 0.2°, 8.1 ± 0.2°, 15.1 ± 0.2° and 17.1 ± 0.2°, preferably further comprising characteristic peaks at 2θ of 19.5 ± 0.2°, 22.7 ± 0.2°, 23.3 ± 0.2° and 30.5 ± 0.2°, more preferably further comprising diffraction peaks at 2θ of 11.5 ± 0.2°, 16.4 ± 0.2°, 19.1 ± 0.2°, 21.9 ± 0.2°, 24.0 ± 0.2°, 25.6 ± 0.2° and 29.5 ± 0.2°; further preferably, the crystal form H has an X-ray powder diffraction pattern substantially as shown in FIG. 8.

The crystal form I of the compound methanesulfonate of formula (I) has an X-ray powder diffraction pattern having a diffraction peak at 2θ of 6.4 ± 0.2°, or having a diffraction peak at 8.4 ± 0.2°, or having a diffraction peak at 9.7 ± 0.2°, or having a diffraction peak at 15.5 ± 0.2°, or having a diffraction peak at 16.8 ± 0.2°, or having a diffraction peak at 17.1 ± 0.2°, or having a diffraction peak at 19.5 ± 0.2°, or having a diffraction peak at 23.6 ± 0.2°, preferably comprising any 2 to 5, or 3 to 5, or 3 to 6, or 3 to 8 of the above diffraction peaks, more preferably comprising any 4, 6, or 8 of the above diffraction peaks.

Preferably, the crystal form I has an X-ray powder diffraction pattern having diffraction peaks at 2θ of 6.4 ± 0.2°, 8.4 ± 0.2°, 9.7 ± 0.2° and 15.5 ± 0.2°, preferably further comprising characteristic peaks at 2θ of 16.8 ± 0.2°, 17.1 ± 0.2°, 19.5 ± 0.2° and 23.6 ± 0.2°, more preferably further comprising diffraction peaks at 2θ of 10.7 ± 0.2°, 13.0 ± 0.2°, 21.7 ± 0.2°, 25.4 ± 0.2°, 25.9 ± 0.2° and 27.2 ± 0.2°; further preferably, the crystal form I has an X-ray powder diffraction pattern substantially as shown in FIG. 9.

In a preferred embodiment of the present invention, the method for preparing the crystal form A of the compound methanesulfonate of formula (I) comprises the steps of:
1) dispersing a free base of the compound of formula (I) in an acetonitrile solvent under a certain temperature condition to form a suspension;
2) adding methanesulfonic acid;
3) continuing to stir, and performing filtration and drying to obtain the crystal form A of the compound methanesulfonate of formula (I),
wherein
in step 1), the temperature condition is 1°C to 60°C, preferably 5°C to 30°C, and the solid-to-liquid ratio of free base to acetonitrile is preferably 0.2 to 2 g : 30 mL, preferably 1 to 1.5 g : 30 mL;
in step 2), the amount of the methanesulfonic acid is preferably 1 to 6 times, preferably 2 to 4 times the molar amount of free base;
in step 3), the stirring time is 3 to 30 h, preferably 5 to 24 h.

In a preferred embodiment of the present invention, the method for preparing the crystal form B of the compound methanesulfonate of formula (I) comprises the steps of:
1) dispersing the compound of formula (I) in a dichloromethane solvent under a certain temperature condition to form a suspension;
2) adding methanesulfonic acid;
3) continuing to stir, and performing filtration and drying to obtain the crystal form B of the compound methanesulfonate of formula (I),
wherein
in step 1), the temperature condition is 1°C to 60°C, preferably 5°C to 30°C, and the solid-to-liquid ratio of the free base to dichloromethane is preferably 0.2 to 2 g : 30 mL, preferably 1 to 1.5 g : 30 mL;
in step 2), the amount of the methanesulfonic acid is preferably 1 to 3 times, preferably 1 to 2 times the molar amount of the free base;
in step 3), the stirring time is 3 to 30 h, preferably 5 to 24 h.

In a preferred embodiment of the present invention, the method for preparing the crystal form C of the compound methanesulfonate of formula (I) comprises the steps of:
1) dissolving the compound of formula (I) in an acetic acid solvent under a certain temperature condition to form a suspension;
2) adding an anti-solvent 2-butanone solvent;
3) continuing to stir, and performing filtration and drying to obtain the crystal form C of the compound methanesulfonate of formula (I),
wherein
in step 1), the temperature condition is 30°C to 80°C, preferably 40°C to 60°C, and the solid-to-liquid ratio of the compound of formula (I) to acetic acid is preferably 0.2 to 2 g : 30 mL, preferably 1 to 1.5 g : 30 mL;
in step 2), the amount of the 2-butanone is preferably 30 to 100 mL, preferably 50 to 80 mL;
in step 3), the stirring time is 1 to 7 days, preferably 2 to 5 days.

In a preferred embodiment of the present invention, the method for preparing the crystal form D of the compound methanesulfonate of formula (I) comprises the steps of:
1) dispersing a free base of the compound of formula (I) in an organic solvent under a certain temperature condition to form a suspension;
2) adding methanesulfonic acid;
3) continuing to stir to precipitate a solid, and performing filtration and drying to obtain the crystal form D of the compound methanesulfonate of formula (I),
wherein
in step 1), the organic solvent is selected from one or more of acetonitrile, ethyl acetate, n-propyl acetate, isopropyl acetate, preferably acetonitrile and ethyl acetate, the temperature condition is 30°C to 90°C, preferably 40°C to 60°C, and the solid-to-liquid ratio of the free base to acetonitrile is preferably 0.2 to 2 g : 30 mL, preferably 1 to 1.5 g : 30 mL;
in step 2), the amount of the methanesulfonic acid is preferably 1 to 3 times, preferably 1 to 2 times the molar amount of the free base;
in step 3), the stirring time is 3 to 30 h, preferably 5 to 24 h.

In a preferred embodiment of the present invention, the method for preparing the crystal form E of the compound methanesulfonate of formula (I) comprises the steps of:
1) dispersing a free base of the compound of formula (I) in an ethyl acetate solvent under a certain temperature condition to form a suspension;
2) adding methanesulfonic acid;
3) continuing to stir to precipitate a solid, and performing filtration and drying to obtain the crystal form E of the compound methanesulfonate of formula (I),
wherein
in step 1), the temperature condition is -30°C to 10°C, preferably -10°C to 0°C, and the solid-to-liquid ratio of the compound of formula I to ethyl acetate is preferably 0.2 to 2 g : 30 mL, preferably 1 to 1.5 g : 30 mL;
in step 2), the amount of the methanesulfonic acid is preferably 1 to 3 times, preferably 1 to 2 times the molar amount of the free base;
in step 3), the stirring time is 3 to 30 h, preferably 5 to 24 h.

In a preferred embodiment of the present invention, the method for preparing the crystal form F of the compound methanesulfonate of formula (I) comprises the steps of:
1) dispersing a free base of the compound of formula (I) in a n-propanol solvent under a certain temperature condition to form a suspension;
2) adding methanesulfonic acid;
3) continuing to stir to precipitate a solid, and performing filtration and drying to obtain the crystal form F of the compound methanesulfonate of formula (I),
wherein
in step 1), the temperature condition is -30°C to 10°C, preferably -10°C to 0°C, and the solid-to-liquid ratio of the compound of formula (I) to n-propanol is preferably 0.2 to 2 g : 30 mL, preferably 1 to 1.5 g : 30 mL;
in step 2), the amount of the methanesulfonic acid is preferably 1 to 3 times, preferably 1 to 2 times the molar amount of the free base;
in step 3), the stirring time is 3 to 30 h, preferably 5 to 24 h.

In a preferred embodiment of the present invention, the method for preparing the crystal form G of the compound methanesulfonate of formula (I) comprises the steps of:
1) dispersing or dissolving the compound of formula (I) in a mixed solvent of an organic solvent and water to form a suspension;
2) continuing to stir the suspension, or adding seed crystals at a certain ratio to induce crystallization;
3) continuing to stir, and performing filtration and drying to obtain a target product,

wherein the organic solvent is selected from one or more of methanol, ethanol, *n*-propanol, *n*-butanol, isopropanol, ethyl acetate, ethylene glycol methyl ether, ethylene glycol ethyl ether, isopropyl ether, dichloromethane, 1,4-dioxane, tetrahydrofuran, acetonitrile, acetone, 2-butanone and water, preferably one or more of 1,4-dioxane, acetonitrile, acetone, 2-butanone or water.
wherein
in step 1), the temperature condition is 10°C to 80°C, preferably 20°C to 50°C, and the ratio of the organic solvent to the water is 1 to 6 : 1, preferably 3 to 6 : 1;
in step 2), the compound of formula (I) is slurried in a mixed solvent of an organic solvent and water for 3 to 5 weeks to obtain seed crystals of the crystal form G, wherein the seed crystals are added at a ratio of 1% to 5%, preferably 1% to 3%;
in step 3), the stirring time is 1 to 7 days, preferably 2 to 5 days.

In a preferred embodiment of the present invention, the method for preparing the crystal form H of the compound methanesulfonate of formula (I) comprises the steps of:
1) dispersing a free base of the compound of formula (I) in an acetonitrile solvent under a certain temperature condition to form a suspension;
2) adding methanesulfonic acid;
3) continuing to stir, and performing filtration and drying to obtain the crystal form H of the compound methanesulfonate of formula (I),
wherein
in step 1), the temperature condition is 1°C to 60°C, preferably 5°C to 30°C, and the solid-to-liquid ratio of free base to acetonitrile is preferably 0.2 to 2 g : 30 mL, preferably 1 to 1.5 g : 30 mL;
in step 2), the amount of the methanesulfonic acid is preferably 1 to 3 times, preferably 1 to 2 times the molar amount of the free base;
in step 3), the stirring time is 1 to 7 days, preferably 2 to 5 days.

In a preferred embodiment of the present invention, the method for preparing the crystal form I of the compound methanesulfonate of formula (I) comprises the steps of:
1) under a certain temperature condition, leaving the crystal form H of the compound methanesulfonate of formula (I) to stand under atmospheric humidity conditions;
2) obtaining the crystal form I of the compound methanesulfonate of formula (I) after standing for a period of time,
wherein
in step 1), the temperature condition is 1°C to 60°C, preferably 5°C to 30°C;
in step 3), the time is 1 to 30 h, preferably 3 to 10 h.

It should be noted that those of ordinary skill in the art should understand that modifications or equivalent substitutions may be made to the technical solutions of the present invention, for example, using some organic solvents exemplified in the foregoing sections of the present invention also falling within the spirit and scope of the technical solutions of the present invention, which should be encompassed within the scope of the present invention.

Another aspect of the present invention provides a pharmaceutical composition, which comprises a therapeutically effective amount of the crystalline free base of the aforementioned compound of formula (I) or the aforementioned crystalline acid salt, or a pharmaceutically acceptable carrier or excipient.

Another aspect of the present invention provides the use of the aforementioned crystalline acid salt of the aforementioned compound of formula (I), the polymorph of the aforementioned acid salt, and the aforementioned pharmaceutical composition in the preparation of a medicament for treating a disease associated with a protein kinase, wherein the protein kinase is selected from c-Met and VEGFR receptor tyrosine kinases.

Another aspect of the present invention provides the use of the aforementioned crystalline acid salt of the aforementioned compound of formula (I), the polymorph of the aforementioned acid salt, and the aforementioned pharmaceutical composition in the preparation of a protein kinase inhibitor, wherein the protein kinase is selected from c-Met and VEGFR receptor tyrosine kinases.

Another aspect of the present invention provides a method for modulating the catalytic activity of a protein kinase, which method comprises bringing the protein kinase into contact with the aforementioned crystalline acid salt of the aforementioned compound of formula (I), the polymorph of the aforementioned acid salt, and the aforementioned pharmaceutical composition, wherein the protein kinase is selected from c-met and VEGFR receptor tyrosine kinases.

The aforementioned crystalline acid salt of the aforementioned compound of formula (I), the polymorph of the aforementioned acid salt, and the aforementioned pharmaceutical composition of the present invention may also be used in the preparation of a medicament for treating cancer and metastases, including cancer (solid tumours), hematopoietic tumours of the lymphatic system, hematopoietic tumours of the myeloid system, tumours of mesenchymal origin, tumours of the central and peripheral nervous systems or other tumours, including but not limited to: the cancer is selected from bladder cancer, breast cancer, colon cancer, kidney cancer, liver cancer, stomach cancer, lung cancer (non-small cell lung cancer) or skin cancer; the hematopoietic tumour of the lymphatic system is selected from leukaemia, acute lymphocytic leukaemia or chronic lymphocytic leukaemia; the hematopoietic tumour of the myeloid system is selected from acute or chronic myeloid leukaemia, myelodysplastic syndrome or promyelocytic leukaemia; the tumour of mesenchymal origin is selected from fibrosarcoma, rhabdomyosarcoma, soft tissue sarcoma or osteosarcoma; the tumour of the central and peripheral nervous systems is selected from astrocytoma, neuroblastoma, glioma or terminal neuroma; the other tumours are selected from malignant melanoma, seminoma, teratocarcinoma, folicullar carcinoma of the thyroid or Kaposi's sarcoma.

The use in the preparation of a medicament for treating liver cancer, lung cancer, breast cancer, epidermal squamous cancer or stomach cancer is preferred.

The use in the preparation of a medicament for treating non-small cell lung cancer is further preferred.

The term "pharmaceutical composition" denotes a mixture containing one or more compounds described herein or physiologically/pharmaceutically acceptable salts or prodrug thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

Different terms such as "X is selected from A, B or C", "X is selected from A, B and C", "X is A, B or C", and "X is A, B and C" all express the same meaning, that is, X can be any one or more of A, B, C.

The crystal form of the compound methanesulfonate of formula (I) of the present invention has good solubility and stability, which enhances its bioavailability, facilitates its pharmaceutical processing and its use in pharmaceutical compositions, and the preparation method of the present invention is simple and easy to implement, suitable for industrial production.

### Brief Description of the Drawings

FIG. 1 is an X-ray diffraction pattern of the crystal form A of the compound methanesulfonate of formula (I).
FIG. 1A is a proton nuclear magnetic resonance spectrum of the crystal form A of the compound methanesulfonate of formula (I).
FIG. 2 is an X-ray diffraction pattern of the crystal form B of the compound methanesulfonate of formula (I).
FIG. 3 is an X-ray diffraction pattern of the crystal form C of the compound methanesulfonate of formula (I).
FIG. 3A is a DSC pattern of the crystal form C of the compound methanesulfonate of formula (I).
FIG. 3B is a TGA pattern of the crystal form C of the compound methanesulfonate of formula (I).
FIG. 4 is an X-ray diffraction pattern of the crystal form D of the compound methanesulfonate of formula (I).
FIG. 4A is a DSC pattern of the crystal form D of the compound methanesulfonate of formula (I).
FIG. 4B is a TGA pattern of the crystal form D of the compound methanesulfonate of formula (I).
FIG. 5 is an X-ray diffraction pattern of the crystal form E of the compound methanesulfonate of formula (I).
FIG. 6 is an X-ray diffraction pattern of the crystal form F of the compound methanesulfonate of formula (I).
FIG. 7 is an X-ray diffraction pattern of the crystal form G of the compound methanesulfonate of formula (I).
FIG. 7A is a proton nuclear magnetic resonance of the crystal form G of the compound methanesulfonate of formula (I).
FIG. 7B is a proton nuclear magnetic resonance of the free base of the compound of formula (I).
FIG. 8 is an X-ray diffraction pattern of the crystal form H of the compound methanesulfonate of formula (I).
FIG. 9 is an X-ray diffraction pattern of the crystal form I of the compound methanesulfonate of formula (I).

### Detailed Description of Embodiments

Specific embodiments of the present invention will be described in further detail below with reference to the accompanying drawings and examples. The following examples are intended to illustrate the present invention, but not to limit the scope of the present invention.

### Example 1: Preparation of crystal form A

1 g of sample, i.e., the free base of the compound of formula (I), was dispersed in 30 mL of acetonitrile solvent at room temperature of 25°C to obtain a suspension, and 3.2 eq of methanesulfonic acid was slowly added dropwise to the base solution. The resulting mixture was stirred for 24 h, filtered, and then subjected to forced-air drying at 50°C for 24 h to obtain the crystal form A of the compound methanesulfonate of formula I.

### Example 2: Preparation of crystal form A

1 g of sample, i.e., the free base of the compound of formula (I), was dispersed in 30 mL of a mixed solvent of acetonitrile and water at room temperature of 25°C to obtain a suspension, and 3.2 eq of methanesulfonic acid was slowly added dropwise to the base solution. The resulting mixture was stirred for 24 h, filtered, and then subjected to forced-air drying at 50°C for 24 h to obtain the crystal form A of the compound methanesulfonate of formula (I).

### Example 3: Preparation of crystal form B

1 g of sample, i.e., the free base of the compound of formula (I), was dispersed in 30 mL of dichloromethane solvent at room temperature of 25°C to obtain a suspension, and 1.2 eq of methanesulfonic acid was slowly added dropwise to the base solution. The resulting mixture was stirred for 24 h, filtered, and then subjected to forced-air drying at 50°C for 24 h to obtain the crystal form B of the compound methanesulfonate of formula (I).

### Example 4: Preparation of crystal form B

1 g of sample, i.e., the free base of the compound of formula (I), was dispersed in 30 mL of dichloromethane solvent at 10°C to obtain a suspension, and 1.2 eq of methanesulfonic acid was slowly added dropwise to the base solution. The resulting mixture was stirred for 24 h, filtered, and then subjected to forced-air drying at 50°C for 24 h to obtain the crystal form B of the compound methanesulfonate of formula (I).

### Example 5: Preparation of crystal form C

1 g of sample, i.e., the compound methanesulfonate of formula (I), was dissolved in 20 mL of acetic acid solvent at room temperature of 25°C to obtain a solution, and 40 mL of anti-solvent *n*-heptane solvent was added to precipitate a solid. The resulting mixture was stirred for 2 days, filtered, and then subjected to forced-air drying at 50°C for 24 h to obtain the crystal form C of the compound methanesulfonate of formula (I).

### Example 6: Preparation of crystal form C

1 g of sample, i.e., the compound methanesulfonate of formula (I) was dissolved in 20 mL of acetic acid solvent at 50°C to obtain a solution, and 40 mL of anti-solvent 2-butanone solvent was added to precipitate a solid. The resulting mixture was stirred for 2 days, filtered, and then subjected to forced-air drying at 50°C for 24 h to obtain the crystal form C of the compound methanesulfonate of formula (I).

### Example 7: Preparation of crystal form D

1 g of sample, i.e., the free base of the compound of formula (I), was dispersed in 30 mL of acetonitrile solvent at 50°C to obtain a suspension, and 1.2 eq of methanesulfonic acid was slowly added dropwise to the base solution. The resulting mixture was stirred for 24 h, filtered, and then subjected to forced-air drying at 50°C for 24 h to obtain the crystal form D of the compound methanesulfonate of formula (I).

### Example 8: Preparation of crystal form D

1 g of sample, i.e., the free base of the compound of formula (I), was dispersed in 30 mL of ethyl acetate solvent at 50°C to obtain a suspension, and 1.2 eq of methanesulfonic acid was slowly added dropwise to the base solution. The resulting mixture was stirred for 24 h, filtered, and then subjected to forced-air drying at 50°C for 24 h to obtain the crystal form D of the compound methanesulfonate of formula (I).

### Example 9: Preparation of crystal form E

1 g of sample, i.e., the free base of the compound of formula (I), was dispersed in 30 mL of ethyl acetate solvent at -10°C to obtain a suspension, and 1.2 eq of methanesulfonic acid was slowly added dropwise to the base solution. The resulting mixture was stirred for 24 h, filtered, and then subjected to forced-air drying at 50°C for 12 h to obtain the crystal form E of the compound methanesulfonate of formula (I).

### Example 10: Preparation of crystal form E

1 g of sample, i.e., the free base of the compound of formula (I), was dispersed in 30 mL of isopropyl acetate solvent at -10°C to obtain a suspension, and 1.2 eq of methanesulfonic acid was slowly added dropwise to the base solution. The resulting mixture was stirred for 24 h, filtered, and then subjected to forced-air drying at 50°C for 24 h to obtain the crystal form E of the compound methanesulfonate of formula (I).

### Example 11: Preparation of crystal form F

1 g of sample, i.e., the free base of the compound of formula (I), was dispersed in 30 mL of n-butanol solvent at -10°C to obtain a suspension, and 1.2 eq of methanesulfonic acid was slowly added dropwise to the base solution. The resulting mixture was stirred for 24 h, filtered, and then subjected to forced-air drying at 50°C for 24 h to obtain the crystal form F of the compound methanesulfonate of formula (I).

### Example 12: Preparation of crystal form F

1 g of sample, i.e., the free base of the compound of formula (I), was dispersed in 30 mL of *n*-propanol solvent at -10°C to obtain a suspension, and 1.2 eq of methanesulfonic acid was slowly added dropwise to the base solution. The resulting mixture was stirred for 24 h, filtered, and then subjected to forced-air drying at 50°C for 24 h to obtain the crystal form F of the compound methanesulfonate of formula (I).

### Example 20: Preparation of crystal form G

1 g of sample, i.e., the compound methanesulfonate of formula (I), was dispersed in 30 mL of a mixed solvent of acetonitrile and water (3 : 1) at room temperature of 25°C to obtain a suspension, and seed crystals of the crystal form G were added at a ratio of 1% to induce crystallization. The resulting mixture was slurried for 1 day, filtered, and then subjected to forced-air drying at 50°C for 24 h to obtain the crystal form G of the compound methanesulfonate of formula (I).

### Example 21: Preparation of crystal form G

1 g of sample, i.e., the compound methanesulfonate of formula (I), was dispersed in 30 mL of a mixed solvent of acetone and water (3 : 1) at room temperature of 25°C to obtain a suspension, and seed crystals of the crystal form G were added at a ratio of 1% to induce crystallization. The resulting mixture was slurried for 3 days, filtered, and then subjected to forced-air drying at 50°C for 24 h to obtain the crystal form G of the compound methanesulfonate of formula (I).

### Example 22: Preparation of crystal form G

1 g of sample, i.e., the compound methanesulfonate of formula (I), was dispersed in 30 mL of a mixed solvent of 2-butanone and water (3 : 1) at 50°C to obtain a suspension, and seed crystals of the crystal form G were added at a ratio of 2% to induce crystallization. The resulting mixture was slurried for 1 day, filtered, and then subjected to forced-air drying at 50°C for 24 h to obtain the crystal form G of the compound methanesulfonate of formula (I).

### Example 23: Preparation of crystal form G

1 g of sample, i.e., the compound methanesulfonate of formula (I), was dispersed in 30 mL of a mixed solvent of acetone and water (3 : 1) at 50°C to obtain a suspension. The resulting mixture was slurried for 3 days, filtered, and then subjected to forced-air drying at 50°C for 24 h to obtain the crystal form G of the compound methanesulfonate of formula (I).

### Example 24: Preparation of crystal form G

1 g of sample, i.e., the compound methanesulfonate of formula (I), was dispersed in 30 mL of a mixed solvent of acetone and water (2 : 1) at room temperature of 25°C to obtain a suspension. The resulting mixture was slurried for 3 days, filtered, and then subjected to forced-air drying at 50°C for 24 h to obtain the crystal form G of the compound methanesulfonate of formula (I).

### Example 25: Preparation of crystal form H

1 g of sample, i.e., the free base of the compound of formula (I), was dispersed in 30 mL of acetonitrile solvent at room temperature of 25°C to obtain a suspension, and 3.2 eq of methanesulfonic acid was slowly added dropwise to the base solution. The resulting mixture was stirred for 2 days, filtered, and then subjected to forced-air drying at 50°C for 24 h to obtain the crystal form H of the compound methanesulfonate of formula (I).

### Example 26: Preparation of crystal form H

1 g of sample, i.e., the free base of the compound of formula (I), was dispersed in 30 mL of a mixed solvent of acetonitrile and water at room temperature of 25°C to obtain a suspension, and 3.2 eq of methanesulfonic acid was slowly added dropwise to the base solution. The resulting mixture was stirred for 5 days, filtered, and then subjected to forced-air drying at 50°C for 24 h to obtain the crystal form H of the compound methanesulfonate of formula (I).

### Example 27: Preparation of crystal form I

1 g of sample, i.e., the crystal form H of the compound methanesulfonate of formula (I) was spread in a watch glass at room temperature of 25°C and left to stand for 3 h to obtain the crystal form I of the compound methanesulfonate of formula (I).

### Example 28: Preparation of crystal form I

1 g of sample, i.e., the crystal form H of the compound methanesulfonate of formula (I) was spread in a watch glass, placed under high humidity conditions of 92.5% humidity at room temperature of 25°C, and left to stand for 10 h to obtain the crystal form I of the compound methanesulfonate of formula (I).

### Experimental example 1: Stability of crystal form G

A sample, i.e., the crystal form G of the compound methanesulfonate of formula (I), was subjected to 1-month stability investigation under conditions of illumination (5000 lux), high temperature of 60°C, and high humidity RH 92.5%. The results showed that the crystal form G was very stable under high temperature, illumination and high humidity conditions, with substantially no chemical degradation occurred under the three conditions. The stability data are as shown in the table below:

| **Test condition** | **Time** | **Crystal form** | **Purity** |
|---|---|---|---|
| **Illumination (5000 lux)** | 0 days | Crystal form G of compound methanesulfonate of formula (I) | 99.5% |
| | 5 days | Crystal form G of compound methanesulfonate of formula (I) | 99.4% |
| | 10 days | Crystal form G of compound methanesulfonate of formula (I) | 99.4% |
| | 30 days | Crystal form G of compound methanesulfonate of formula (I) | 99.4% |
| **High temperature (60°C)** | 0 days | Crystal form G of compound methanesulfonate of formula (I) | 99.5% |
| | 5 days | Crystal form G of compound methanesulfonate of formula (I) | 99.5% |
| | 10 days | Crystal form G of compound methanesulfonate of formula (I) | 99.4% |
| | 30 days | Crystal form G of compound methanesulfonate of formula (I) | 99.4% |
| **High humidity (RH 92.5%)** | 0 days | Crystal form G of compound methanesulfonate of formula (I) | 99.5% |
| | 5 days | Crystal form G of compound methanesulfonate of formula (I) | 99.5% |
| | 10 days | Crystal form G of compound methanesulfonate of formula (I) | 99.5% |
| | 30 days | Crystal form G of compound methanesulfonate of formula (I) | 99.4% |

### Experimental example 2: Stability of crystal form G under high humidity condition

The crystal form G of the compound methanesulfonate of formula (I) was subjected to 3-month long-term stability investigation under high humidity RH 92.5% condition. The results showed that the crystal form G was very stable under high humidity condition, with no polymorphic transformation and dissociation occurred.

Finally, it should be noted that the above examples are only used to illustrate the technical solutions of the present invention and not to limit the present invention. Although the present invention has been described in detail with reference to the preferred examples, those of ordinary skill in the art should understand that modifications or equivalent substitutions may be made to the technical solutions of the present invention without departing from the spirit and scope of the technical solutions of the present invention, which shall be covered by the claims of the present invention.

## Claims

1. A crystal form G of 9-((8-fluoro-6-(1-methyl-1*H*-pyrazol-4-yl)-[1,2,4]triazolo[4,3-*a*]pyridin-3-yl)-thio)-4methyl-2*H*-[1,4]oxazino[3,2-*c*]quinoline-3(4H)-one methanesulfonate, **characterized in that**
the crystal form G has an X-ray powder diffraction pattern having a diffraction peak at 2θ of 8.5 ± 0.2°, or having a diffraction peak at 9.0 ± 0.2°, or having a diffraction peak at 9.4 ± 0.2°, or having a diffraction peak at 9.8 ± 0.2°, or having a diffraction peak at 16.9 ± 0.2°, or having a diffraction peak at 19.7 ± 0.2°, or having a diffraction peak at 22.7 ± 0.2°, or having a diffraction peak at 25.5 ± 0.2°, or having a diffraction peak at 26.2 ± 0.2°, or having a diffraction peak at 31.8 ± 0.2°, or having a diffraction peak at 32.5 ± 0.2°, preferably comprising any 2 to 5, or 3 to 5, or 3 to 6, or 3 to 8 of the above diffraction peaks, more preferably comprising any 4, 6, or 8 of the above diffraction peaks.

2. The crystal form G of methanesulfonate according to claim 1, **characterized in that**
the crystal form G has an X-ray powder diffraction pattern having diffraction peaks at 2θ of 9.8 ± 0.2° and 16.9 ± 0.2°, preferably further comprising characteristic peaks at 2θ of 8.5 ± 0.2° and 9.0 ± 0.2°, preferably further comprising characteristic peaks at 2θ of 19.7 ± 0.2°, 22.7 ± 0.2°, 25.5 ± 0.2° and 26.2 ± 0.2°, more preferably further comprising diffraction peaks at 2θ of 9.4 ± 0.2°, 12.4 ± 0.2°, 13.0 ± 0.2°, 24.8 ± 0.2°, 27.4 ± 0.2°, 28.1 ± 0.2°, 31.8 ± 0.2° and 32.5 ± 0.2°; further preferably, the crystal form G has an X-ray powder diffraction pattern substantially as shown in FIG. 7.

3. Crystal forms A-F, H and I of 9-((8-fluoro-6-(1-methyl-1*H*-pyrazol-4-yl)-[1,2,4]triazolo[4,3-*a*]pyridin-3-yl)-thio)-4methyl-2*H*-[1,4]oxazino[3,2-*c*]quinoline-3(4*H*)-one methanesulfonate, **characterized in that**
the crystal form A has an X-ray powder diffraction pattern having a diffraction peak at 2θ of 5.4 ± 0.2°, or having a diffraction peak at 6.1 ± 0.2°, or having a diffraction peak at 10.6 ± 0.2°, or having a diffraction peak at 13.7 ± 0.2°, or having a diffraction peak at 18.1 ± 0.2°, or having a diffraction peak at 19.6 ± 0.2°, or having a diffraction peak at 24.7 ± 0.2°, preferably comprising any 2 to 5, or 3 to 5, or 3 to 6, or 3 to 7 of the above diffraction peaks, more preferably comprising any 4, 6, or 7 of the above diffraction peaks;
the crystal form B has an X-ray powder diffraction pattern having a diffraction peak at 2θ of 5.4 ± 0.2°, or having a diffraction peak at 7.8 ± 0.2°, or having a diffraction peak at 10.6 ± 0.2°, or having a diffraction peak at 13.7 ± 0.2°, or having a diffraction peak at 14.4 ± 0.2°, or having a diffraction peak at 15.6 ± 0.2°, or having a diffraction peak at 19.7 ± 0.2°, preferably comprising any 2 to 5, or 3 to 5, or 3 to 6, or 3 to 7 of the above diffraction peaks, more preferably comprising any 4, 6, or 7 of the above diffraction peaks
the crystal form C has an X-ray powder diffraction pattern having a diffraction peak at 2θ of 7.8 ± 0.2°, or having a diffraction peak at 9.6 ± 0.2°, or having a diffraction peak at 11.7 ± 0.2°, or having a diffraction peak at 15.6 ± 0.2°, or having a diffraction peak at 17.7 ± 0.2°, or having a diffraction peak at 23.4 ± 0.2, or having a diffraction peak at 26.5 ± 0.2, preferably comprising any 2 to 5, or 3 to 5, or 3 to 6, or 3 to 7 of the above diffraction peaks, more preferably comprising any 4, 6, or 7 of the above diffraction peaks;
the crystal form D has an X-ray powder diffraction pattern having a diffraction peak at 2θ of 5.2 ± 0.2°, or having a diffraction peak at 11.5 ± 0.2°, or having a diffraction peak at 13.5 ± 0.2°, or having a diffraction peak at 17.1 ± 0.2°, or having a diffraction peak at 19.8 ± 0.2, or having a diffraction peak at 22.8 ± 0.2, or having a diffraction peak at 23.3 ± 0.2, or having a diffraction peak at 24.2 ± 0.2, preferably comprising any 2 to 5, or 3 to 5, or 3 to 6, or 3 to 8 of the above diffraction peaks, more preferably comprising any 4, 6, or 8 of the above diffraction peaks;
the crystal form E has an X-ray powder diffraction pattern having a diffraction peak at 2θ of 6.4 ± 0.2°, or having a diffraction peak at 7.8 ± 0.2°, or having a diffraction peak at 9.7 ± 0.2°, or having a diffraction peak at 10.7 ± 0.2°, or having a diffraction peak at 12.9 ± 0.2°, or having a diffraction peak at 15.4 ± 0.2°, or having a diffraction peak at 16.8 ± 0.2°, or having a diffraction peak at 19.5 ± 0.2°, preferably comprising any 2 to 5, or 3 to 5, or 3 to 6, or 3 to 8 of the above diffraction peaks, more preferably comprising any 4, 6, or 8 of the above diffraction peaks;
the crystal form F has an X-ray powder diffraction pattern having a diffraction peak at 2θ of 5.4 ± 0.2°, or having a diffraction peak at 6.5 ± 0.2°, or having a diffraction peak at 8.1 ± 0.2°, or having a diffraction peak at 9.7 ± 0.2°, or having a diffraction peak at 13.3 ± 0.2°, or having a diffraction peak at 15.5 ± 0.2°, or having a diffraction peak at 16.9 ± 0.2°, or having a diffraction peak at 21.6 ± 0.2°, preferably comprising any 2 to 5, or 3 to 5, or 3 to 6, or 3 to 8 of the above diffraction peaks, more preferably comprising any 4, 6, or 8 of the above diffraction peaks;
the crystal form H has an X-ray powder diffraction pattern having a diffraction peak at 2θ of 7.5 ± 0.2°, or having a diffraction peak at 8.1 ± 0.2°, or having a diffraction peak at 15.1 ± 0.2°, or having a diffraction peak at 17.1 ± 0.2°, or having a diffraction peak at 19.5 ± 0.2°, or having a diffraction peak at 22.7 ± 0.2°, or having a diffraction peak at 23.3 ± 0.2°, or having a diffraction peak at 30.5 ± 0.2°, preferably comprising any 2 to 5, or 3 to 5, or 3 to 6, or 3 to 8 of the above diffraction peaks, more preferably comprising any 4, 6, or 8 of the above diffraction peaks;
the crystal form I has an X-ray powder diffraction pattern having a diffraction peak at 2θ of 6.4 ± 0.2°, or having a diffraction peak at 8.4 ± 0.2°, or having a diffraction peak at 9.7 ± 0.2°, or having a diffraction peak at 15.5 ± 0.2°, or having a diffraction peak at 16.8 ± 0.2°, or having a diffraction peak at 17.1 ± 0.2°, or having a diffraction peak at 19.5 ± 0.2°, or having a diffraction peak at 23.6 ± 0.2°, preferably comprising any 2 to 5, or 3 to 5, or 3 to 6, or 3 to 8 of the above diffraction peaks, more preferably comprising any 4, 6, or 8 of the above diffraction peaks.

4. The crystal forms A-F, H and I of methanesulfonate according to claim 3, **characterized in that**
the crystal form A has an X-ray powder diffraction pattern having diffraction peaks at 2θ of 5.4 ± 0.2°, 6.1 ± 0.2°, 10.6 ± 0.2° and 13.7 ± 0.2°, preferably further comprising diffraction peaks at 2θ of 18.1 ± 0.2°, 19.6 ± 0.2°, and 24.7 ± 0.2°, more preferably further comprising diffraction peaks at 2θ of 8.7 ± 0.2°, 11.7 ± 0.2°, 14.8 ± 0.2°, 17.4 ± 0.2°, 21.1 ± 0.2° and 27.1 ± 0.2°; further preferably, the crystal form A has an X-ray powder diffraction pattern substantially as shown in FIG. 1;
the crystal form B has an X-ray powder diffraction pattern having diffraction peaks at 2θ of 5.4 ± 0.2°, 7.8 ± 0.2°, 10.6 ± 0.2° and 13.7 ± 0.2°, preferably further comprising characteristic peaks at 2θ of 14.4 ± 0.2, 15.6 ± 0.2 and 19.7 ± 0.2, more preferably further comprising diffraction peaks at 2θ of 4.9 ± 0.2°, 8.7 ± 0.2°, 9.7 ± 0.2°, 11.5 ± 0.2°, 17.6 ± 0.2°, 24.4 ± 0.2°, 25.2 ± 0.2° and 27.6 ± 0.2°; further preferably, the crystal form B has an X-ray powder diffraction pattern substantially as shown in FIG. 2;
the crystal form C has an X-ray powder diffraction pattern having diffraction peaks at 2θ of 7.8 ± 0.2°, 9.6 ± 0.2°, 11.7 ± 0.2° and 15.6 ± 0.2°, preferably further comprising characteristic peaks at 2θ of 17.7 ± 0.2, 23.4 ± 0.2 and 26.5 ± 0.2, more preferably further comprising diffraction peaks at 2θ of 11.3 ± 0.2°, 12.4 ± 0.2°, 19.1 ± 0.2°, 21.9 ± 0.2°, 24.6 ± 0.2°, 25.1 ± 0.2°, 29.4 ± 0.2°, 31.3 ± 0.2° and 34.2 ± 0.2°; further preferably, the crystal form C has an X-ray powder diffraction pattern substantially as shown in FIG. 3;
the crystal form D has an X-ray powder diffraction pattern having diffraction peaks at 2θ of 5.2 ± 0.2°, 11.5 ± 0.2°, 13.5 ± 0.2° and 17.1 ± 0.2°, preferably further comprising characteristic peaks at 2θ of 19.8 ± 0.2, 22.8 ± 0.2, 23.3 ± 0.2 and 24.2 ± 0.2, more preferably further comprising diffraction peaks at 2θ of 8.2 ± 0.2°, 8.6 ± 0.2°, 10.5 ± 0.2°, 14.6 ± 0.2°, 16.4 ± 0.2°, 20.8 ± 0.2°, 21.7 ± 0.2°, 25.7 ± 0.2°, 29.4 ± 0.2° and 30.6 ± 0.2°; further preferably, the crystal form D has an X-ray powder diffraction pattern substantially as shown in FIG. 4;
the crystal form E has an X-ray powder diffraction pattern having diffraction peaks at 2θ of 6.4 ± 0.2°, 7.8 ± 0.2°, 9.7 ± 0.2° and 10.7 ± 0.2°, preferably further comprising characteristic peaks at 2θ of 12.9 ± 0.2, 15.4 ± 0.2, 16.8 ± 0.2 and 19.5 ± 0.2, more preferably further comprising diffraction peaks at 2θ of 11.1 ± 0.2°, 14.1 ± 0.2°, 18.1 ± 0.2°, 21.6 ± 0.2°, 23.5 ± 0.2°, 25.4 ± 0.2° and 25.9 ± 0.2°; further preferably, the crystal form E has an X-ray powder diffraction pattern substantially as shown in FIG. 5;
the crystal form F has an X-ray powder diffraction pattern having diffraction peaks at 2θ of 5.4 ± 0.2°, 6.5 ± 0.2°, 8.1 ± 0.2° and 9.7 ± 0.2°, preferably further comprising characteristic peaks at 2θ of 13.3 ± 0.2°, 15.5 ± 0.2°, 16.9 ± 0.2° and 21.6 ± 0.2°, more preferably further comprising diffraction peaks at 2θ of 10.8 ± 0.2°, 13.0 ± 0.2°, 18.2 ± 0.2°, 18.9 ± 0.2°, 19.6 ± 0.2°, 22.3 ± 0.2°, 23.7 ± 0.2° and 24.4 ± 0.2°; further preferably, the crystal form F has an X-ray powder diffraction pattern substantially as shown in FIG. 6;
the crystal form H has an X-ray powder diffraction pattern having diffraction peaks at 2θ of 7.5 ± 0.2°, 8.1 ± 0.2°, 15.1 ± 0.2° and 17.1 ± 0.2°, preferably further comprising characteristic peaks at 2θ of 19.5 ± 0.2°, 22.7 ± 0.2°, 23.3 ± 0.2° and 30.5 ± 0.2°, more preferably further comprising diffraction peaks at 2θ of 11.5 ± 0.2°, 16.4 ± 0.2°, 19.1 ± 0.2°, 21.9 ± 0.2°, 24.0 ± 0.2°, 25.6 ± 0.2° and 29.5 ± 0.2°;
further preferably, the crystal form H has an X-ray powder diffraction pattern substantially as shown in FIG. 8;
the crystal form I has an X-ray powder diffraction pattern having diffraction peaks at 2θ of 6.4 ± 0.2°, 8.4 ± 0.2°, 9.7 ± 0.2° and 15.5 ± 0.2°, preferably further comprising characteristic peaks at 2θ of 16.8 ± 0.2°, 17.1 ± 0.2°, 19.5 ± 0.2° and 23.6 ± 0.2°, more preferably further comprising diffraction peaks at 2θ of 10.7 ± 0.2°, 13.0 ± 0.2°, 21.7 ± 0.2°, 25.4 ± 0.2°, 25.9 ± 0.2° and 27.2 ± 0.2°; further preferably, the crystal form I has an X-ray powder diffraction pattern substantially as shown in FIG. 9.

5. A method for preparing the crystal form G of 9-((8-fluoro-6-(1-methyl-1*H*-pyrazol-4-yl)-[1,2,4]triazolo[4,3-*a*]pyridin-3-yl)-thio)-4-methyl-2*H-*[1,4]oxazino[3,2-*c*]quinoline-3(4*H*)-one methanesulfonate according to claims 1 to 2, **characterized by** comprising the steps of:
1) dispersing or dissolving the compound methanesulfonate of formula (I) in a mixed solvent consisting of an organic solvent and water to form a suspension;
2) continuing to stir the above suspension, or adding seed crystals at a certain ratio to induce crystallization;
3) continuing to stir, and performing filtration and drying to obtain a target product.

6. The method for preparing the crystal form G of methanesulfonate according to claim 5, **characterized in that** in step 1), the organic solvent is selected from one or more of methanol, ethanol, *n*-propanol, *n*-butanol, isopropanol, ethyl acetate, ethylene glycol methyl ether, ethylene glycol ethyl ether, isopropyl ether, dichloromethane, 1,4-dioxane, tetrahydrofuran, acetonitrile, acetone, 2-butanone or water, preferably one or more of 1,4-dioxane, acetonitrile, acetone, 2-butanone or water.

7. The method for preparing the crystal form G of methanesulfonate according to claim 5, **characterized in that** in step 1), the temperature condition is - 30°C to 80°C, preferably 0°C to 50°C, and the ratio of the organic solvent to the water is 1 to 6 : 1, preferably 3 to 6 : 1; in step 2), the seed crystals are added at a ratio of 1% to 5%, preferably 1% to 3%; in step 3), the stirring time is 1 to 7 days, preferably 2 to 5 days.

8. A pharmaceutical composition, **characterized by** comprising a therapeutically effective amount of the 9-((8-fluoro-6-(1-methyl-1H-pyrazol-4-yl)-[1,2,4]triazolo[4,3-a]pyridin-3-yl)-thio)-4-methyl-2H-[1,4]oxazino[3,2-c]quinoline-3(4H)-one crystalline acid salt or a polymorph thereof according to claims 1-4, and one or more pharmaceutically acceptable carriers or excipients.

9. Use of the 9-((8-fluoro-6-(1-methyl-1H-pyrazol-4-yl)-[1,2,4]triazolo[4,3-a]pyridin-3-yl)-thio)-4-methyl-2H-[1,4]oxazino[3,2-c]quinoline-3(4H)-one crystalline acid salt or a polymorph thereof according to claims 1 to 4 and the pharmaceutical composition according to claim 8 in the preparation of a medicament for treating a disease associated with a protein kinase, wherein preferably, the protein kinase is selected from c-Met and VEGFR receptor tyrosine kinases.

10. Use of the 9-((8-fluoro-6-(1-methyl-1H-pyrazol-4-yl)-[1,2,4]triazolo[4,3-a]pyridin-3-yl)-thio)-4-methyl-2H-[1,4]oxazino[3,2-c]quinoline-3(4H)-one crystalline acid salt or a polymorph thereof according to claims 1 to 4 and the pharmaceutical composition according to claim 8 in the preparation of a medicament for treating cancer and metastases, including cancer, hematopoietic tumours of the lymphatic system, hematopoietic tumours of the myeloid system, tumours of mesenchymal origin, tumours of the central and peripheral nervous systems or other tumours;
preferably, the cancer is selected from bladder cancer, breast cancer, colon cancer, kidney cancer, liver cancer, lung cancer, stomach cancer or skin cancer; the hematopoietic tumour of the lymphatic system is selected from leukaemia, acute lymphocytic leukaemia or chronic lymphocytic leukaemia; the hematopoietic tumour of the myeloid system is selected from acute or chronic myeloid leukaemia, myelodysplastic syndrome or promyelocytic leukaemia; the tumour of mesenchymal origin is selected from fibrosarcoma, rhabdomyosarcoma, soft tissue sarcoma or osteosarcoma; the tumour of the central and peripheral nervous systems is selected from astrocytoma, neuroblastoma, glioma or terminal neuroma; the other tumours are selected from malignant melanoma, seminoma, teratocarcinoma, folicullar carcinoma of the thyroid or Kaposi's sarcoma; further preferably, the cancer is non-small cell lung cancer.
